(19) Europäisches Patentamt
European Patent Office
Office européen des brevets

(11) EP 0 910 549 B1

(12) **FASCICULE DE BREVET EUROPEEN**

(45) Date de publication et mention de la délivrance du brevet:
**17.10.2001 Bulletin 2001/42**

(21) Numéro de dépôt: **97931875.5**

(22) Date de dépôt: **04.07.1997**

(51) Int Cl.[7]: **C01G 23/053**, C09C 1/36, C09D 5/32

(86) Numéro de dépôt international:
**PCT/FR97/01208**

(87) Numéro de publication internationale:
**WO 98/01392 (15.01.1998 Gazette 1998/02)**

(54) **PARTICULES DE DIOXYDE DE TITANE, LEUR PROCEDE DE PREPARATION ET LEUR UTILISATION DANS LES COSMETIQUES, VERNIS ET LASURES**

TITANDIOXIDTEILCHEN, IHR HERSTELLUNGSVERFAHREN UND IHRE VERWENDUNG IN KOSMETIKA UND LACKEN

TITANIUM DIOXIDE PARTICLES, METHOD FOR THEIR PREPARATION AND THEIR USE IN COSMETICS, VARNISH AND SURFACE COATING

(84) Etats contractants désignés:
**AT BE CH DE DK ES FI FR GB GR IE IT LI LU NL PT SE**

(30) Priorité: **08.07.1996 FR 9608460**
**27.09.1996 FR 9611781**

(43) Date de publication de la demande:
**28.04.1999 Bulletin 1999/17**

(73) Titulaire: **RHODIA CHIMIE**
**92408 Courbevoie Cédex (FR)**

(72) Inventeurs:
- **CHOPIN, Thierry**
**F-95320 Saint-Leu la Forêt (FR)**
- **DUPUIS, Dominique**
**F-95170 Deuil-la-Barre (FR)**
- **PACAUD, Bernard**
**F-78100 Saint-Germain en Laye (FR)**

(74) Mandataire: **Ducreux Bertrand, Marie et al**
**Rhodia Services**
**Propriété Industrielle,**
**25, Quai Paul Doumer**
**92408 Courbevoie Cedex (FR)**

(56) Documents cités:

| | |
|---|---|
| **EP-A- 0 401 045** | **WO-A-95/12638** |
| **DE-A- 4 302 896** | **GB-A- 2 115 394** |
| **US-A- 4 461 810** | **US-A- 4 923 682** |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouvelles particules de dioxyde de titane anatase présentant des propriétés anti-UV, utilisables notamment dans les formulations cosmétiques, les vernis et les lasures.
Il est connu d'utiliser le dioxyde de titane en tant qu'agent anti-UV dans de nombreuses applications en particulier en cosmétique, peinture, plastique, ...
Dans ces applications, le dioxyde de titane se présente généralement sous forme de particules de taille inférieure à 100 nm en dispersion dans une phase aqueuse ou organique.
Le problème issu de l'utilisation de ces dispersions de particules de dioxyde de titane tient au fait que ces dernières sont souvent instables. Pour contrôler leur stabilité, il est connu de leur ajouter des agents dispersants, en général des polymères organiques.
Cependant, l'ajout de ces agents dispersants n'est pas une solution sans inconvénient. En effet, lorsque la dispersion de dioxyde de titane est mélangée à d'autres produits pour préparer une formulation en cosmétique, peinture ou plastique, cet agent peut présenter des incompatibilités avec l'application (stabilité, agglomération, toxicité, ...) ou les autres composants de la formule.
Dans ces applications, le besoin se fait donc sentir de pouvoir disposer de dispersions de particules de dioxyde de titane stables sans ajout d'agent dispersant.
Un but de la présente invention est donc de proposer des dispersions de particules de dioxyde de titane stables ne contenant pas d'agent dispersant.
Dans ce but, l'invention concerne des particules de dioxyde de titane recouvertes au moins partiellement :

- d'une première couche d'au moins un composé du cérium et/ou du fer, et
- d'une deuxième couche d'au moins un oxyde, hydroxyde ou oxohydroxyde métallique,

lesdites particules présentant une surface spécifique BET d'au moins 70 $m^2/g$ et une densité de l'ordre de 2,5.
Dans ce but, l'invention concerne également le procédé de préparation de ces particules dans lequel on met en oeuvre les étapes suivantes :

- on précipite au moins un composé du cérium et/ou du fer à la surface de particules de dioxyde de titane anatase de taille d'au plus 100 nm, présentant une surface spécifique BET d'au moins 200 $m^2/g$ et une densité de l'ordre de 2,5, puis
- on précipite au moins un oxyde, hydroxyde ou oxohydroxyde métallique à la surface de particules obtenues.

Enfin, l'invention concerne aussi l'utilisation de ces particules en tant qu'agent anti-UV dans les formulations pour cosmétiques, peintures, vemis, lasures et dans les plastiques ; et plus particulièrement une composition cosmétique anti-UV et une composition de lasure anti-UV.
Les dispersions de particules selon l'invention présentent en outre l'avantage d'être stables sur une large gamme de pH sans ajout d'agent dispersant.
Elles peuvent également présentées des extraits secs élevés tout en restant stables et en présentant une viscosité faible, notamment inférieure à 1000 mPa.s.
De plus, on observe que ces dispersions conservent le même indice de dispersion même lorsqu'elles ont été mélangées avec les composants d'une formulation par exemple cosmétique.
D'autres caractéristiques, détails et avantages de l'invention apparaîtront plus clairement à la lecture de la description et des exemples qui vont suivre.
L'invention concerne tout d'abord des particules de dioxyde de titane recouvertes au moins partiellement :

- d'une première couche d'au moins un composé du cérium et/ou du fer, et
- d'une deuxième couche d'au moins un oxyde, hydroxyde ou oxohydroxyde métallique,

lesdites particules présentant une surface spécifique BET d'au moins 70 $m^2/g$ et une densité de l'ordre de 2,5.
Les particules selon l'invention sont à base de dioxyde de titane de structure cristalline majoritairement anatase. "Majoritairement" signifie que le taux d'anatase des particules de dioxyde de titane du revêtement est supérieur à 50 % en masse. De préférence, les particules du revêtement présentent un taux d'anatase supérieur à 80 %. Le taux de cristallisation et la nature de la phase cristalline sont mesurés par diffraction RX.
Le diamètre moyen de ces particules est d'au plus 100 nm, de préférence d'au moins 25 nm, encore plus préférentiellement comprise entre 50 et 70 nm. Ce diamètre est mesuré par microscopie électronique par transmission (MET).
Les particules selon l'invention présentent une surface spécifique BET d'au moins 70 $m^2/g$, de préférence

d'au moins 100 $m^2/g$.

On entend par surface spécifique BET, la surface spécifique déterminée par adsorption d'azote conformément à la norme ASTMD 3663-78 établie à partir de la méthode BRUNAUER - EMMETT - TELLER décrite dans le périodique "The Journal of the American Society", 60, 309 (1938). Pour mesurer la surface spécifique des particules selon l'invention, lorsqu'elles se présentent sous forme de dispersion, il est essentiel de suivre le protocole de mesure qui consiste à éliminer la phase liquide de la dispersion puis à sécher les particules sous vide à une température de 150°C pendant au moins 4 heures.

Les particules selon l'invention présentent également une densité de l'ordre de 2,5. Par "de l'ordre", on entend que la densité est de 2,5 ± 0,2. Une telle valeur de densité est faible par rapport à la densité classique du dioxyde de titane anatase qui est de 3,8. Cette densité est mesurée par picnométrie.

Les particules selon l'invention sont recouvertes au moins partiellement d'une première couche minérale à base d'au moins un composé du cérium et/ou du fer. Ces composés sont des précurseurs de l'oxyde de cérium ou de fer, c'est-à-dire qu'ils sont thermiquement décomposables en oxyde de cérium ou fer. Il peut s'agir, en général, de sels de cérium ou de fer.

Selon l'invention, les particules recouvertes d'un composé du cérium sont préférées. Les meilleurs résultats en propriétés de dispersion ont été obtenus pour les particules dont le rapport en poids du ou des composés du cérium sur le dioxyde de titane est d'au plus 6 % en poids, exprimée en $CeO_2$.

Ce rapport peut être optimisé en fonction de la taille des particules. Ainsi, il a été observé que pour des particules de diamètre 25 nm, la teneur optimale en cérium était de 5,5 % en poids, exprimée en $CeO_2$, par rapport au dioxyde de titane, de même pour des particules de diamètre 45 nm, cette teneur est de 4,5 %, pour des particules de diamètre 60 nm, cette teneur est de 3 %, et pour des particules de diamètre 80 nm, cette teneur est de 2 %.

Ces particules sont également recouvertes au moins partiellement d'une deuxième couche à base d'au moins un oxyde, hydroxyde ou oxohydroxyde métallique. L'oxyde est, en général, $SiO_2$, quant à l'hydroxyde ou oxohydroxyde métallique, il peut être en particulier choisi parmi les hydroxydes ou oxohydroxydes de l'aluminium, de zinc, de titane ou d'étain sous forme simple ou mixte. Par mixte, on entend un composé métallique à base d'au moins deux des éléments précités (silicoaluminates, ...).

En général, le rapport en poids du ou des oxydes, hydroxydes ou oxohydroxydes métalliques sur le dioxyde de titane est d'au plus 60 % en poids. Ce rapport est fonction de l'application à laquelle les particules sont destinées. De préférence, lorsque les particules sont utilisées en application cosmétique, ce rapport est d'au plus 25 %, encore plus préférentiellement d'au plus 20 %.

Ces quantités de composés, oxydes, hydroxydes ou oxohydroxydes métalliques sont mesurées sur les particules en dispersion par fluorescence X.

Selon le mode préféré de l'invention, les particules sont recouvertes au moins partiellement d'une première couche d'un composé du cérium et d'une deuxième couche à base de silice et/ou d'un hydroxyde ou oxohydroxyde d'aluminium sous forme simple ou mixte.

Selon une première variante préférée, les particules sont recouvertes d'une deuxième couche à base de silice et d'hydroxyde ou oxohydroxyde d'aluminium dans des teneurs en poids de 15 % de $SiO_2$ et 5 % d'$Al_2O_3$ par rapport au dioxyde de titane.

Selon une deuxième variante encore plus préférée, les particules sont recouvertes d'une deuxième couche à base uniquement de silice dans une teneur en poids de 30 % de $SiO_2$.

Selon le mode préféré de l'invention, les particules se présentent sous la forme d'une dispersion.

Cette dispersion peut présenter une proportion de solide en suspension (extrait sec) comprise entre 10 et 60 % en poids, de préférence d'au moins 35 %, encore plus préférentiellement d'au moins 40 %.

Cette dispersion présente, en général, un indice de dispersion des particules dans la phase liquide d'au plus 0,5. L'indice de dispersion est déterminé par la formule :

$$I = \frac{\varnothing_{84} - \varnothing_{16}}{2\varnothing_{50}}$$

dans laquelle :

- $\varnothing_{84}$ est le diamètre des particules pour lequel 84% des particules ont un diamètre inférieur à $\varnothing_{84}$,
- $\varnothing_{16}$ est le diamètre des particules pour lequel 16% des particules ont un diamètre inférieur à $\varnothing_{16}$,
- $\varnothing_{50}$ est le diamètre moyen des particules.

Les diamètres utiles à la détermination de l'indice de dispersion sont mesurés par sédimentation centrifuge des particules de la dispersion, suivie par rayons X, à l'aide d'un appareil Brookhaven type XDC.

Un tel indice traduit la bonne dispersibilité des particules. Dans le cas des dispersions aqueuses, cet indice est obtenu sur une large gamme de pH pouvant varier de 5,5 à 10. Les dispersions sont stables, elles conservent cette valeur d'indice dans le temps, malgré l'absence d'agent dispersant.

Selon un premier mode, cette dispersion est en phase aqueuse.

En général, cette dispersion aqueuse présente une conductivité d'au plus 3 msiemens.

Les dispersion de particules selon l'invention présentant un extrait sec d'au moins 35 % ont l'avantage d'être peu visqueuses, ainsi leur viscosité est en général d'au plus 1000 mPa.s.

Selon un deuxième mode, cette dispersion est en phase organique. Cette dernière est en général obtenue à partir d'une dispersion aqueuse et transfert des particules en phase organique.

Par exemple, ce transfert peut être réalisé par greffage d'une chaîne hydrophobe, par exemple un trialcoxysilane, à la surface des particules en dispersion aqueuse. La dispersion de particules compatibilisées est ensuite mélangée à un milieu organique de manière à faire migrer les particules dans la phase organique.

La dispersion organique peut aussi être obtenue par mise en contact d'une dispersion aqueuse de particules de dioxyde de titane avec le solvant organique désiré puis chauffage de manière à éliminer l'eau par distillation. Ce dernier procédé ne peut être mis en oeuvre que dans le cas où le solvant organique choisi présente une température d'ébullition supérieure à celle de l'eau et est soluble dans l'eau.

Un autre procédé consiste à mélanger une dispersion aqueuse avec un milieu organique comprenant un agent de transfert cationique, ce dernier pouvant être choisi, par exemple, parmi les amines quaternaires ou les sels d'ammonium quaternaire, ce procédé est plus particulièrement décrit dans le brevet GB-A-988.330.

Enfin, ces particules selon l'invention peuvent également être agglomérées et se présenter sous forme d'une poudre. La taille des agglomérats peut être comprise entre 1 et 40 µm, mesurée par MET.

Suite à un traitement organique, cette poudre peut présenter une bonne redispersibilité en milieu organique. Ce traitement organique peut être réalisé, par exemple, par atomisation en présence d'un acide gras tel que l'acide stéarique, d'un sel métallique d'un acide gras.

L'invention concerne également le procédé de préparation de ces particules qui consiste à mettre en oeuvre les étapes suivantes :

- on précipite au moins un composé du cérium et/ou du fer à la surface de particules de dioxyde de titane anatase de taille d'au plus 100 nm, présentant une surface spécifique BET d'au moins 200 $m^2/g$ et une densité de l'ordre de 2,5, puis
- on précipite au moins un oxyde, hydroxyde ou oxohydroxyde métallique à la surface de particules obtenues.

Ces précipitations peuvent être réalisées par :

. introduction, dans une dispersion de particules de dioxyde de titane présentant les caractéristiques ci-dessus définies, de précurseurs des composés du cérium et/ou du fer, oxydes, hydroxydes ou oxohydroxydes métalliques en général sous forme de solutions aqueuses de sels, puis,

. modification du pH pour obtenir la précipitation de ces composés, oxydes, hydroxydes ou oxohydroxydes sur les particules de dioxyde de titane.

En général, on effectue cette précipitation à une température d'au moins 50°C.

Une caractéristique importante des particules selon l'invention est qu'elles ne sont pas calcinées, c'est-à-dire qu'elles ne sont pas recouvertes d'oxydes de cérium et/ou de fer.

Les composés du cérium et/ou du fer sont en général des sels ou hydroxydes de cérium ou de fer. Pour le cérium, il peut s'agir d'un sel de cérium choisi parmi l'acétate de cérium, le sulfate de cérium ou le chlorure de cérium.

De même, pour le dépôt du fer, il peut s'agir d'un chlorure, d'un sulfate ou d'un acétate de fer.

Selon un mode préférentiel, cette couche est obtenue par précipitation de l'acétate de cérium et/ou du chlorure de fer.

L'utilisation de nitrate de cérium ou de nitrate de fer est fortement déconseillée, car le traitement obtenu peut avoir tendance à conduire à un effet de photo-bleuissement de la formulation dans laquelle la dispersion de dioxyde de titane serait utilisée.

En général, la précipitation des composés du cérium et/ou du fer est obtenue pour un pH compris entre 4 et 10.

Il est possible de chauffer la dispersion de particules au cours de cette étape.

Dans le cas de la précipitation de silice et d'un hydroxyde ou oxohydroxyde d'aluminium, la précipitation peut être réalisée à pH acide ou basique. Le pH est contrôlé par l'ajout d'un acide tel que l'acide sulfurique ou par l'introduction simultanée et/ou alternative d'un composé alcalin du silicium et d'un composé acide de l'aluminium. De préférence, dans ce cas, le pH est compris entre 8 et 10.

On peut précipiter la silice à partir d'un sel de silicium tel qu'un silicate alcalin.

L'hydroxyde ou oxohydroxyde d'aluminium peut être précipité à partir d'un sel d'aluminium tel que le sulfate d'alumine, l'aluminate de soude, le chlorure basique d'aluminium, l'hydroxyde d'aluminium diacétate.

On peut, après la précipitation, récupérer et laver les particules obtenues à la suite du traitement avant de les remettre en dispersion. Cette étape peut être réalisée par centrifugation et lavage ou, de préférence, par lavage par ultrafiltration. Le pH de l'eau de lavage est avantageusement de l'ordre de 5,5. Puis, les particules sont redispersées dans un autre milieu liquide de manière à obtenir une dispersion de particules de dioxyde de titane. Ce milieu liquide peut être acide ou basique, de préférence, il s'agit une solution basique présentant un pH de l'ordre de 8-9.

Pour obtenir une poudre de particules selon l'invention, on sèche la dispersion issue du procédé, en général à une température inférieure à 110°C.

Les particules de dioxyde de titane anatase de départ doivent présenter une taille d'au plus 100 nm, une surface spécifique BET d'au moins 200 $m^2$/g et une densité de l'ordre de 2,5.

Les particules de départ sont à base de dioxyde de titane de structure cristalline majoritairement anatase, ainsi que défini précédemment.

Le diamètre moyen de ces particules est d'au plus 100 nm, de préférence d'au moins 25 nm, encore plus préférentiellement compris entre 50 et 70 nm. Ce diamètre est mesuré par microscopie électronique par transmission (MET).

Les particules de départ présentent une surface spécifique BET d'au moins 200 $m^2$/g, de préférence d'au moins 250 $m^2$/g.

Cette surface spécifique BET est mesurée de la même manière que définie précédemment.

Les particules de départ présentent également une densité de l'ordre de 2,5. Par "de l'ordre", on entend que la densité est de 2,5 ± 0,2. Cette densité est donnée par la formule suivante :

$$\text{densité} = \frac{1}{(1/\varrho) + Vi}$$

dans laquelle :

- $\varrho$ est la densité de l'anatase, soit 3,8,
- Vi est le volume apporté par les pores intra particules, il est mesuré par la méthode BJH. On -entend par volume mesuré par la méthode BJH, la volume mesuré à partir de la méthode BARRETT-JOYNER-HELENDA décrite dans l'article de l'ouvrage Techniques de l'Ingénieur, et intitulé "Texture des solides poreux ou divisés", p.3645-1 à 3645-13.

Pour mesurer le volume apporté par les pores intra particules des particules selon l'invention, lorsqu'elles se présentent sous forme de dispersion, il est essentiel de suivre le protocole de mesure qui consiste à éliminer la phase liquide de la dispersion puis à sécher les particules sous vide à une température de 150°C pendant au moins 4 heures.

De telles particules peuvent être obtenues par hydrolyse d'au moins un composé du titane A en présence d'au moins un composé B choisi parmi :

(i) les acides qui présentent :

- soit un groupement carboxyle et au moins deux groupements hydroxyles et/ou amines,
- soit au moins deux groupements carboxyles et au moins un groupement hydroxyle et/ou amine,

(ii) les acides phosphoriques organiques de formules suivantes :

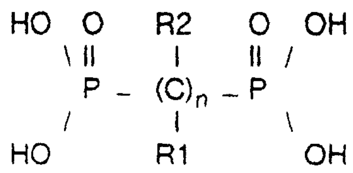

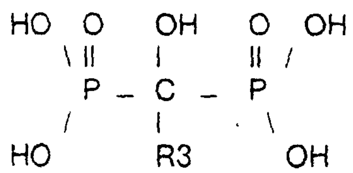

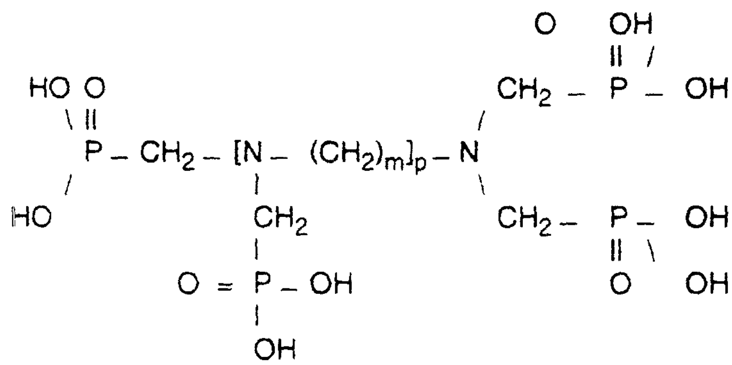

dans lesquelles, n et m sont des nombres entiers compris entre 1 et 6, p est un nombre entier compris entre 0 et 5, R1, R2, R3 identiques ou différents représentant un groupement hydroxyle, amino, aralkyl, aryl, alkyl ou l'hydrogène,

(iii) les composés capables de libérer des ions sulfates en milieu acide,

(iv) les sels des acides décrits ci-dessus,

et en présence de germes de dioxyde de titane anatase présentant une taille d'au plus 8 nm et dans un rapport pondéral exprimé en $TiO_2$ présent dans les germes/titane présent avant introduction des germes dans le milieu d'hydrolyse, exprimé en $TiO_2$, compris entre 0,01 % et 3 %.

La solution de départ, destinée à être hydrolysée, est de préférence totalement aqueuse ; éventuellement on peut ajouter un autre solvant, un alcool par exemple, à condition que le composé du titane A et le composé B utilisés soient alors substantiellement solubles dans ce mélange.

En ce qui concerne le composé du titane A, on utilise en général un composé choisi parmi les halogénures, les oxyhalogénures, les alcoxydes de titane, les sulfates et plus particulièrement les sulfates synthétiques.

On entend par sulfates synthétiques des solutions de sulfates de titanyle réalisées par échange d'ions à partir de solutions de chlorure de titane très pures ou par réaction d'acide sulfurique sur un alcoxyde de titane.

De préférence, on opère avec des composés du titane du type halogénure ou oxyhalogénure de titane. Les halogénures ou les oxyhalogénures de titane plus particulièrement utilisés dans la présente invention sont les fluorures, les chlorures, les bromures et les iodures (respectivement les oxyfluorures, les oxychlorures, les oxybromures et les oxyiodures) de titane.

Selon un mode particulièrement préféré, le composé du titane est l'oxychlorure de titane $TiOCl_2$.

La quantité de composé de titane A présente dans la solution à hydrolyser n'est pas critique.

La solution initiale contient en outre au moins un composé B tel que défini précédemment. A titre d'exemples non limitatifs de composés B entrant dans le cadre de la présente invention, on peut citer notamment :

- les acides hydroxypolycarboxyliques, et plus particulièrement les acides hydroxydi- ou hydroxytricarboxyliques tels que l'acide citrique, l'acide maléique et l'acide tartrique.
- les acides (polyhydroxy)monocarboxyliques, comme par exemple l'acide glucoheptonique et l'acide gluconique,

- les acides poly(hydroxycarboxyliques), comme par exemple l'acide tartrique,
- les monoacides dicarboxyliques et leurs amides correspondantes, comme par exemple l'acide aspartique, l'asparagine et l'acide glutamique,
- les aminoacides monocarboxyliques, hydroxylés ou non, comme par exemple la lysine, la sérine et la thréonine,
- l'aminotriphosphonate de méthylène, l'éthylènediaminotétraphosphonate de méthylène, le triéthylènetétraaminohexaphosphonate de méthylène, le tétraéthylènepentaaminoheptaphosphonate de méthylène, le pentaéthylènehexaaminooctaphosphonate de méthylène,
- le diphosphonate de méthylène; de 1,1' éthylène; de 1,2 éthylène; de 1,1' propylène; de 1,3 propylène; de 1,6 hexaméthylène; le 2,4 dihydroxypentaméthylène - 2,4 diphosphonate; le 2,5 dihydroxyhexaméthylène - 2,5 disphosphonate ; le 2,3 dihydroxybutylène - 2,3 diphosphonate ; le 1 hydroxybenzyle - 1,1' diphosphonate ; le 1 aminoéthylène 1-1' diphosphonate ; l'hydroxyméthylène diphosphonate ; le 1 hydroxyéthylène 1,1' diphosphonate ; le 1 hydroxypropylène 1-1' diphosphonate ; le 1 hydroxybutylène 1-1' diphosphonate ; le 1 hydroxyhexaméthylène - 1,1' diphosphonate.

Comme déjà indiqué, il est également possible d'utiliser à titre de composé B tous les sels des acides précités. En particulier, ces sels sont soit des sels alcalins, et plus particulièrement des sels de sodium, soit des sels d'ammonium.

Ces composés peuvent être choisis aussi parmi l'acide sulfurique et les sulfates d'ammonium, de potassium,...

De préférence, les composés B tels que définis ci-dessus sont des composés hydrocarbonés de type aliphatique. Dans ce cas, la longueur de la chaîne principale hydrocarbonée n'excède pas de préférence 15 atomes de carbone, et plus préférentiellement 10 atomes de carbone. Le composé B préféré est l'acide citrique.

La quantité de composé B n'est pas critique. D'une manière générale, la concentration molaire du composé B par rapport à celle du composé du titane A est comprise entre 0,2 et 10 % et de préférence entre 1 et 5 %.

Enfin la solution de départ comprend des germes de dioxyde de titane utilisés d'une manière spécifique.

Ainsi, les germes de dioxyde de titane utilisés dans la présente invention doivent présenter tout d'abord une taille inférieure à 8 nm, mesurée par diffraction X. De préférence, on utilise des germes de dioxyde de titane présentant une taille comprise entre 3 et 5 nm.

Ensuite, le rapport pondéral du dioxyde de titane présent dans les germes sur le titane présent dans le milieu d'hydrolyse avant introduction des germes - c'est-à-dire apporté par le composé du titane A - et exprimé en $TiO_2$ est compris entre 0,01 et 3 %. Ce rapport peut être préférentiellement compris entre 0,05 et 1,5 %. La réunion de ces deux conditions sur les germes (taille et rapport pondéral) associée au procédé tel que décrit précédemment permet de contrôler précisément la taille finale des particules de dioxyde de titane en associant à un taux de germes une taille de particule. On peut ainsi obtenir des particules dont le diamètre varie entre 25 et 100 nm.

On utilise des germes de dioxyde de titane sous forme anatase de manière à induire la précipitation du dioxyde de titane sous forme anatase. Généralement, du fait de leur petite taille, ces germes se présentent plutôt sous la forme d'anatase mal cristallisé. Les germes se présentent habituellement sous la forme d'une suspension aqueuse constituée de dioxyde de titane. Ils peuvent être généralement obtenus de manière connue par un procédé de neutralisation d'un sel de titane par une base.

L'étape suivante consiste à réaliser l'hydrolyse de cette solution de départ par tout moyen connu de l'homme du métier et en général par chauffage. Dans ce dernier cas, l'hydrolyse peut de préférence être effectuée à une température supérieure ou égale à 70°C. On peut aussi travailler dans un premier temps à une température inférieure à la température d'ébullition du milieu puis maintenir le milieu d'hydrolyse en palier à la température d'ébullition.

Une fois l'hydrolyse réalisée, les particules de dioxyde de titane obtenues sont récupérées par séparation du solide précipité des eaux mères avant d'être redispersées dans un milieu liquide de manière à obtenir une dispersion de dioxyde de titane. Ce milieu liquide peut être acide ou basique. Il s'agit de préférence d'une solution basique, par exemple d'une solution aqueuse de soude. C'est à partir de cette dispersion que l'étape de précipitation des oxydes, hydroxydes ou oxohydroxydes métalliques sera réalisée.

Selon une variante particulière, après la récupération des particules obtenues à la suite de l'hydrolyse et avant leur remise en dispersion, on neutralise les particules et on leur fait subir au moins un lavage. Les particules peuvent être récupérées par exemple par centrifugation de la solution issue de l'hydrolyse, elles sont ensuite neutralisées par une base, par exemple une solution d'ammoniaque ou de soude, puis on les lave en les redispersant dans une solution aqueuse, enfin les particules sont séparées de la phase aqueuse de lavage. Après éventuellement un ou plusieurs autres lavages du même type, les particules sont remises en dispersion dans une solution acide ou basique.

Ces particules présentent habituellement un degré de pureté élevé, compatible avec une application en cosmétique.

Enfin, l'invention concerne l'utilisation des particules précédemment décrites en tant qu'agent anti-UV. Elles peuvent notamment être utilisées en tant qu'agent anti-UV dans les formulations pour cosmétiques, revêtement tels que vernis, peintures, lasures, et dans les plastiques.

Introduites dans les formulations cosmétiques, ces dispersions ou poudres de dioxyde de titane permettent d'obtenir un indice SPF (Sun Protection Factor) d'au moins 20.

D'autre part, les formulations obtenues sont photostables, c'est-à-dire qu'elles ne présentent pas de bleuissement après exposition aux UV selon le test défini dans les exemples.

Elles sont particulièrement stables au stockage et on peut constater que les particules de dioxyde de titane conserve leur indice de dispersion dans la formulation.

L'invention concerne des compositions cosmétiques anti-UV comprenant des particules telles que précédemment décrites, en quantité telle que la teneur en dioxyde de titane dans lesdites compositions est d'au moins 1 %, de préférence d'au plus 25 % en poids, encore plus préférentiellement comprise entre 2 et 10 % en poids.

Il est possible d'introduire dans les compositions cosmétiques des particules présentant des tailles de particules différentes.

Les compositions faisant l'objet de l'invention peuvent être formulées en un grand nombre de types de produits, comme les produits solaires type gels, lotions, huiles, crèmes, et plus généralement les produits de maquillage, les autobronzants, les produits de soin, les capillaires, les écrans totaux pour les lèvres et bien d'autres compositions du même type.

On entend par le terme composition ou formulation cosmétique tous les produits ou préparations cosmétiques comme celles décrites dans l'annexe I ("illustrative list by category of cosmetic products") de la directive européenne n° 76/768/CEE du 27 juillet 1976 dite directive cosmétique.

Les compositions cosmétiques faisant l'objet de l'invention peuvent faire appel à un véhicule, ou un mélange de plusieurs véhicules, qui jouent un rôle de diluant, dispersant ou de support pour les autres constituants de la composition et permettent leur répartition lorsque la composition est étalée sur la peau ou les cheveux.

Les véhicules autres que l'eau peuvent être des émolients liquides ou solides, des solvants, des humectants, des épaississants ou des poudres. On peut par exemple utiliser seuls ou en mélange les types de véhicules suivants :

- des émollients tels que l'alcool stéarylique, le glycéryl monoricinoléate, l'alcool oléylique, l'alcool cétylique, l'isopropyl isostéarate, l'acide stéarique, l'isobutyl palmitate, l'isocétyl stéarate , l'isopropyl laurate, l'hexyl laurate, le décyloléate, l'octadécane-2-ol, l'alcool isocétylique, l'alcool eicosanylique, l'alcool behenylique, le cétylpalmitate, les huiles silicones telles que le diméthylpolysiloxne, le di-n-butyl sebacate, l'isopropyl myristate, l'isopropyl palmitate, l'isopropyl stéarate, le butyl stéarate, le polyéthylène glycol, la lanoline, le beurre de coco, l'huile de graine de coton, l'huile d'olive, l'huile de palme, l'huile de colza, l'huile de soja, l'huile de tournesol, l'huile d'avocat, l'huile d'amande, l'huile de sésame, l'huile de noix de coco, l'huile d'arachide, l'huile de castor, l'huile minérale, le myristate de butyl, l'acide isostéarique, l'acide palmitique, l'isopropyl linoléate, le lauryl lactate, le décyl oléate, le pyristyl myristate ;
- des propulseurs tels que : propane, butane, isobutane, diméthyléther, dioxyde de carbone, dioxyde d'azote ;
- des solvants tels que : éthanol, chlorure de méthylène, isopropanol, acétone, éthylène glycol monoéthyl éther, diéthylène glycol monobutyl éther, diéthylène glycol monoéthyl éther, oxyde de diméthylsulfure, diméthylformamide, tétrahydrofurane ;
- des poudres telles que craie, talc, kaolin, amidon, gommes, silice colloïdale, polyacrylate de sodium, smectites de tétraalkyl et/ou trialkyl aryl ammonium, magnésoaluminosilicate chimiquement modifié, montmorillonite organiquement modifié, silicate d'aluminium hydraté, fumée de silice, polycarboxyvinyl, carboxyméthylcellulose de sodium, éthylène glycol monostéarate.

Les compositions selon l'invention comprennent habituellement de 10 à 99 % en poids d'au moins un véhicule tel que décrit précédemment.

De préférence, les compositions selon l'invention se présentent sous la forme d'émulsions, dans lesquelles un composant huileux est présent avec un émulsifiant de manière à former une émulsion huile dans l'eau ou eau dans l'huile, selon la valeur de la balance hydrophile/lipophile (HLB).

Ainsi, les compositions selon l'invention peuvent comprendre un ou plusieurs composants huileux ou composants ayant les propriétés d'une huile.

Il peut s'agir d'huiles végétales ou minérales, telles que celles proposées dans la liste des émollients cidessus. On peut également utilisés des huiles silicones, volatiles ou non, tels que des polydiméthylsiloxanes.

Ces composants huileux peuvent représenter jusqu'à 90 %, de préférence de 10 à 80 %, du volume de la composition.

Les compositions selon l'invention peuvent comprendre également un ou plusieurs émulsifiants. Selon la nature de ces émulsifiants, les compositions se présenteront sous la forme d'une émulsion huile dans l'eau ou eau dans l'huile.

Pour la préparation d'une émulsion type eau dans l'huile, le ou les émulsifiants choisis doivent présenter une HLB moyenne comprise entre 1 et 6. Pour la préparation d'une émulsion type huile dans l'eau, le ou les émulsifiants

choisis doivent présenter une HLB moyenne comprise supérieure à 6. La quantité de ces émulsifiant dans les compositions selon l'invention peuvent varier entre 1 et 50 % en poids, de préférence entre 2 et 20 %.

Ces compositions cosmétiques peuvent aussi contenir des agents tensioactifs qui servent à disperser, émulsionner, solubiliser, stabiliser divers composés utilisés pour leurs propriétés émollientes ou humectantes. Ces agents tensioactifs sont utilisés dans ces compositions à des concentrations variant de 0,05 à 50 % en poids de la préparation. On retrouve ainsi des tensioactifs anioniques, non-ioniques, cationiques, zwitterioniques ou amphotères ou des mélanges de ces tensioactifs, tels que :

- **des tensioactifs anioniques :**

  . les alkylesters sulfonates de formule R-CH($SO_3$M)-COOR', où R représente un radical alkyle en $C_8$-$C_{20}$, de préférence en $C_{10}$-$C_{16}$, R' un radical alkyle en $C_1$-$C_6$, de préférence en $C_1$-$C_3$ et M un cation alcalin (sodium, potassium, lithium), ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...). On peut citer tout particulièrement les méthyl ester sulfonates dont les radical R est en $C_{14}$-$C_{16}$;

  . les alkylsulfates de formule $ROSO_3M$, où R représente un radical alkyle ou hydroxyalkyle en $C_{10}$-$C_{24}$, de préférence en $C_{12}$-$C_{20}$ et tout particulièrement en $C_{12}$-C18, M représentant un atome d'hydrogène ou un cation de même définition que ci-dessus, ainsi que leurs dérivés éthoxylénés (OE) et/ou propoxylénés (OP), présentant en moyenne de 0,5 à 6 motifs, de préférence de 0,5 à 3 motifs OE et/ou OP ;

  . les alkylamides sulfates de formule RCONHR'$OSO_3$M où R représente un radical alkyle en $C_2$-$C_{22}$, de préférence en $C_6$-$C_{20}$, R' un radical alkyle en $C_2$-$C_3$, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;

  . les sels d'acides gras saturés ou insaturés en $C_8$-$C_{24}$, de préférence en $C_{14}$-$C_{20}$, les alkylbenzènesulfonates en $C_9$-$C_{20}$, les alkylsulfonates primaires ou secondaires en $C_8$-$C_{22}$, les alkylglycérol sulfonates, les acides polycarboxyliques sulfonés décrits dans GB - A - 1 082 179, les sulfonates de paraffine, les N-acyl N-alkyltaurates, les alkylphosphates, les alkyliséthionates, les alkylsuccinamates les alkylsulfosuccinates, les monoesters ou diesters de sulfosuccinates, les N-acyl sarcosinates, les sulfates d'alkylglycosides, les polyéthoxycarboxylates, le cation étant un métal alcalin (sodium, potassium, lithium), un reste ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...) ;

- **des agents tensio-actifs non-ioniques :**

  . les alkylphénols polyoxyalkylénés (polyéthoxyéthylénés, polyoxypropylénés, polyoxybutylénés) dont le substituant alkyle est en $C_6$-$C_{12}$ et contenant de 5 à 25 motifs oxyalkylènes ; à titre d'exemple, on peut citer les TRITON X-45, X-114, X-100 ou X-102 commercialisés par Rohm & Haas Cy. ;

  . les glucosamides, glucamides ;

  . les glycérolamides dérivés de N-alkylamines (US-A-5,223,179 et FR-A-1,585,966)

  . les alcools aliphatiques en $C_8$-$C_{22}$ polyoxyalkylénés contenant de 1 à 25 motifs oxyalkylènes (oxyéthylène, oxypropylène) ; à titre d'exemple, on peut citer les TERGITOL 15-S-9, TERGITOL 24-L-6 NMW commercialisés par Union Carbide Corp., NEODOL 45-9, NEODOL 23-65, NEODOL 45-7, NEODOL 45-4 commercialisés par Shell Chemical Cy., KYRO EOB commercialisé par The Procter & Gamble Cy.

  . les produits résultant de la condensation de l'oxyde d'éthylène avec un composé hydrophobe résultant de la condensation de l'oxyde de propylène avec le propylène glycol, tels les PLURONIC commercialisés par BASF ;

  . les oxydes d'amines tels que les oxydes d'alkyl $C_{10}$-$C_{18}$ diméthylamines, les oxydes d'alkoxy $C_8$-$C_{22}$ éthyl dihydroxy éthylamines ;

  . les alkylpolyglycosides décrits dans US-A-4 565 647 et leurs polyoxyalkylénés;

  . les amides d'acides gras en $C_8$-$C_{20}$

  . les acides gras éthoxylés

  . les amides, amines, amidoamines éthoxylées

- **des agents tensio-actifs amphotères et zwitterioniques :**

  . les alkyltriméthylsulfobétaïnes, les produits de condensation d'acides gras et d'hydrolysats de proteines, les alkylampho-propionates ou -dipropionates, les alkylsultaines, les dérivés amphotères des alkylpolyamines comme l'AMPHIONIC XL® commercialisé par RHONE-POULENC, AMPHOLAC 7T/X® et AMPHOLAC 7C/X® commercialisés par BEROL NOBEL sont utilisés pour diminuer l'irritation provoquée par les autres agents tensioactifs, principalement les agents tensioactifs anioniques.

On peut également utiliser un émulsifiant choisi parmi ceux de la liste suivante :

| Nom chimique de l'émulsifiant | Nom commercial | HLB |
|---|---|---|
| trioléate sorbitan | Arlacel 85 | 1,8 |
| tristéarate sorbitan | Span 65 | 2,1 |
| glycerol monooléate | Aldo MD | 2,7 |
| glycerol monostéarate | Atmul 84S | 2,8 |
| glycerol monolaurate | Aldo MC | 3,3 |
| sesquioleate sorbitan | Arlacel 83 | 3,7 |
| monooleate sorbitan | Arlacel 80 | 4,3 |
| monostéarate sorbitan | Arlacel 60 | 4,7 |
| polyoxyéthylène stéaryl éther | Brij 72 | 4,9 |
| polyoxyéthylène sorbitol dérivé de cire d'abeille | G-1702 | 5 |
| polyglyceryl-3 diisostéarate | Plurol Diisostéarique | 6 |
| PEG 200 dilaurate | Emerest 2622 | 6,3 |
| monopalmitate sorbitan | Arlacel 40 | 6,7 |
| PEG 200 monostéarate | Tegester PEG 200 MS | 8,5 |
| sorbitan monolaurate | Arlacel 200 | 8,6 |
| PEG 400 dioléate | Tegester PEG 400-DO | 8,8 |
| polyoxyéthylène (5) monostéarate | Ethofat 60-16 | 9,0 |
| polyoxyéthylène (4) sorbitan monostéarate | Tween 61 | 9,6 |
| polyoxyéthylène (4) lauryléther | Brij 30 | 9,7 |
| polyoxyéthylène (5) sorbitan monooléate | Tween 81 | 10,0 |
| PEG 300 monooléate | Neutronyx 834 | 10,4 |
| polyoxyéthylène (20) sorbitan tristéarate | Tween 65 | 10,5 |
| polyoxyéthylène (20) sorbitan trioléate | Tween 85 | 11,0 |
| polyoxyéthylène monostéarate | Myrj 45 | 11,1 |
| PEG 400 monooléate | Emerest 2646 | 11,7 |
| PEG 400 monostéarate | Tegester PEG 400 | 11,9 |
| polyoxyéthylène 10 monooléate | Ethofat 0/20 | 12,2 |
| polyoxyéthylène 10 stéaryl éther | Brij 76 | 12,4 |
| polyoxyéthylène 10 cétyl éther | Brij 56 | 12,9 |
| polyoxyéthylène (4) sorbitan monolaurate | Tween 21 | 13,3 |
| PEG 600 monooléate | Emerest 2660 | 13,7 |
| PEG 1000 dilaurate | Kessco | 13,9 |
| polyoxyéthylène sorbitol dérivé de la lanoline | G-1441 | 14,0 |
| polyoxyéthylène (12) lauryl éther | Ethosperse LA-12 | 14,4 |
| PEG 1500 dioléate | Pegosperse 1500 | 14,6 |
| polyoxyéthylène (14) laurate | Arosurf HFL-714 | 14,8 |
| polyoxyéthylène (20) sorbitan monostéarate | Tween | 14,9 |
| polyoxyéthylène (20) sorbitan monooléate | Tween 80 | 15,0 |
| polyoxyéthylène (20) stéaryl éther | Brij 78 | 15,3 |
| polyoxyéthylène (20) sorbitan monopalmitate | Tween 40 | 15,6 |
| polyoxyéthylène (20) cétyl éther | Brij 58 | 15,7 |
| polyoxyéthylène (25) oxypropylène | Monostéarate G-2162 | 16,0 |
| polyoxyéthylène (20) sorbitol monolaurate | Tween 20 | 16,7 |
| polyoxyéthylène (23) lauryl éther | Brij 35 | 16,9 |
| polyoxyéthylène (50) monostéarate | Myrj 53 | 17,9 |

(suite)

| Nom chimique de l'émulsifiant | Nom commercial | HLB |
|---|---|---|
| PEG 4000 monostéarate | Pegoperse 4000 MS | 18,7 |

Les compositions selon l'invention peuvent comprendre de l'eau dans une teneur pouvant aller jusqu'à 80 % en volume, de préférence comprise entre 5 et 80 %.

Les compositions selon l'invention peuvent comprendre en outre un agent tensio-actif siliconé de haut poids moléculaire, qui peut être un émulsifiant utilisé à la place de ceux mentionnés ci-dessus.

Cet agent peut être un diméthylpolysiloxane de haut poids moléculaire avec des chaînes polyoxyéthylène et/ou polyoxypropylène ayant un poids moléculaire compris entre 10 000 et 50 000 et de structure :

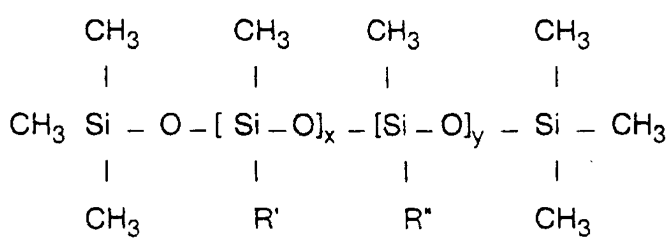

, dans laquelle :

- les groupes R' et R" sont choisis parmi H, les alkyl en $C_1$-$C_{18}$ et $[CH_2CH_2O]_a[CH_2CH(CH_3)O]_bH$, l'un des groupes R' et R" peut être le lauryl, l'autre ayant un poids moléculaire sompris entre 1000 et 5000,
- a est compris entre 9 et 115, de préférence entre 10 et 114
- b est compris entre 0 et 50, de préférence entre 0 et 49,
- x est compris entre 133 et 673, de préférence entre 388 et 402
- y est compris entre 25 et 0,25, de préférence entre 15 et 0,75.

Le diméthylpolysiloxane peut être utilisé sous la forme d'une dispersion dans un siloxane volatile, cette dispersion comprenant de 1 à 20 % en volume de diméthylpolysiloxane.

Les diméthylpolysiloxanes peuvent être choisis parmi les cyclométhicone et diméthicone coplyols, tels que DC 3225C de Dow Corning ou le lauryl méthicone copolyol tel que le DC Q2-5200 de Dow Corning.

Il peut s'agir aussi du Mirasil DMCO de Rhône-Poulenc, du cetyl dimethicone copylol Abil AM90 de TH. Goldschmidt AG.

La composition selon l'invention peut comprendre jusqu'à 25 % en poids d'un tel agent tensio-actif.

Les compositions selon l'invention peuvent comprendre également un filtre solaire organique, tel que par exemple

| Nom CTFA | Nom commercial | Commersialisé par |
|---|---|---|
| Benzophénone-3 | UVINUL M-40 | BASF |
| Benzophénone-4 | UVINUL MS-40 | BASF |
| Benzophénone-8 | SPECRA-SORB UV-24 | American Cyanamid |
| Glyceryl PABA | NIPA GMPA | Nipa Labs |
| Octocrylène | UVINUL N-539 SG | BASF |
| Octyl diméthyl PABA | ESCALOL 507 | ISP |
| Octyl méthoxycinnamate | PARSOL MCX | Givaudan/Roux |
| Octyl salicylate | UVINUL O-18 | BASF |
| PABA | n° 102 | Merck |
| Acide sulfonique 2-phénylbenzimidazole-5 | EUSOLEX 232 | Merck |
| 3-(4-méthylbenzylidène)-Camphre | EUSOLEX 6300 | EM Ind. |
| 4-isopropyl dibenzoyl methane | EUSOLEX 8020 | EM Ind. |

(suite)

| Nom CTFA | Nom commercial | Commersialisé par |
|---|---|---|
| Butyl méthoxy dibenzoyl methane | PARSOL 1789 | Givaudan/Roux |
| Etocrylène | UVINUL N-35 | BASF |

On peut également utiliser en tant que filtre solaire tous les composés autorisés dans la directive Européenne N° 76/768/CEE, et ses annexes.

La composition selon l'invention peut comprendre également des agents solaires inorganiques tels que : de l'oxyde de zinc sous forme de particules de taille moyenne compris entre 1 et 300 nm, de l'oxyde de fer sous forme de particules de taille moyenne compris entre 1 et 300 nm, de la silice sous forme de particules de taille moyenne compris entre 1 et 100 nm.

Les compositions peuvent comprendre aussi des addtitifs tels que :

- des conservateurs par exemple les ester de para-hydroxy-benzoate ;
- des antioxydants tels que le butylhydroxytoluène ;
- des humectants tels que le glycérol, le sorbitol, le dibutylphtalate, la gélatine, les PEG par exemple les PEG 200-600 ;
- des solutions tampons telles que des mélanges d'acide lactique et de soude ou de triéthanolamine ;
- des cires telles que la cire d'abeille, de paraffine ;
- des extraits de plantes ;
- des agents conservateurs comme les méthyl, éthyl, propyl et butyl esters de l'acide p-hydroxybenzoïque, le benzoate de sodium, le GERMABEN (nom de marque) ou tout agent chimique évitant la prolifération bactérienne ou des moississures et utilisé traditionnellement des les compositions cosmétiques sont généralement introduits dans ces compositions à hauteur de 0,01 à 3 % en poids. La quantité de ces produits est généralement ajustée pour éviter toute prolifération de bactéries, moississures ou levures dans les compositions cosmétiques. Alternativement à ces agents chimiques, on peut parfois utiliser des agents modifiants l'activité de l'eau et augmentant fortement la pression osmotique comme les carbohydrates ou des sels ;
- ...

Les compositions cosmétiques faisant l'objet de l'invention peuvent également contenir des résines fixatives. Ces résines fixatives sont généralement présentes à des concentrations comprises entre 0.01 et 10%, préférentiellement entre 0,5 et 5 %. Les résines fixatives constituants des compositions cosmétiques faisant l'objet de l'invention sont préférentiellement choisies parmi les résines suivantes : acrylate/acrylamide copolymère, polyvinyl méthyl éther/ maléic anhydride copolymère, vinyl acétate/ acide crotonique copolymère, octylacrylamide/acrylate/butylaminoéthyl méthacrylate copolymère, polyvinylpyrrolidone (PVP), copolymères de polyvinylpyrrolidone et de méthyl méthacrylate, copolymère de polyvinylpyrrolidone et d'acétate de vinyle (VA), alccol polyvinylique, copolymère de l'alcool polyvinylique et d'acide crotonique, copolymère d'alcool polyvinylique et d'anhydride maléique, hydroxypropyl cellulose, hydroypropyl guar, polystyrène sulfonate de sodium, polyvinylpyrrolidone/éthyl méthacrylate/ acide méthacrylique terpolymère, monométhyl éther de poly(méthylvinyl éther - acide maléique), les copolymères polytéréphtale d'éthylène glycol / polyéthylène glycol, les copolymères polytéréphtalate d'éthylène glycol / polyéthylène glycol / polyisophtalate sulfonate de sodium, et leurs mélanges. Les résines fixatives peuvent aussi contenir des motifs polyorganosiloxanes fonctionnalisés greffés comme décrit dans le brevet WO 95/06079.

De manière préférentielle, les résines fixatives seront du type polyvinylpyrrolidone (PVP), copolymères de polyvinylpyrrolidone et de méthyl méthacrylate, copolymère de polyvinylpyrrolidone et d'acétate de vinyle (VA), copolymères polytéréphtale d'éthylène glycol / polyéthylène glycol, copolymères polytéréphtalate d'éthylène glycol / polyéthylène glycol / polyisophtalate sulfonate de sodium, et leurs mélanges.

Ces résines fixatives sont préférentiellement dispersées ou solubilisées dans le véhicule choisi.

Les compositions cosmétiques faisant l'objet de l'invention peuvent également contenir des dérivés polymériques exerçant une fonction protectrice.

Ces dérivés polymériques peuvent être présents en quantités de l'ordre de 0,01 à 10 %, de préférence environ 0,1 à 5 %, et tout particulièrement de l'ordre de 0,2 à 3 % en poids, agents tels que

. les dérivés cellulosiques tels que les hydroxyéthers de cellulose, la méthylcellulose, l'éthylcellulose, l'hydroxypropyl méthylcellulose, l'hydroxybutyl méthylcellulose

. les polyvinylesters greffés sur des troncs polyalkylenes tels que les polyvinylacétates greffés sur des troncs polyoxyéthylènes (EP-A-219 048)

- les alcools polyvinyliques
- les copolymères polyesters à base de motifs ethylène téréphtalate et/ou propylène téréphtalate et polyoxyéthylène téréphtalate, avec un rapport molaire (nombre de motifs) ethylène téréphtalate et/ou propylène téréphalate / (nombre de motifs) polyoxyéthylène téréphtalate de l'ordre de 1/10 à 10/1, de préférence de l'ordre de 1/1 à 9/1, les polyoxyéthylène téréphtalates présentant des unités polyoxyéthylène ayant un poids moléculaire de l'ordre de 300 à 5000, de préférence de l'ordre de 600 à 5000 (US-A-3 959 230, US-A-3 893 929, US-A-4 116 896, US-A-4 702 857, US-A-4 770 666) ;
- les oligomères polyesters sulfonés obtenus par sulfonation d'un oligomère dérivé de de l'alcool allylique éthoxylé, du diméthyltéréphtalate et du 1,2 propylène diol, présentant de 1 à 4 groupes sulfonés (US-A-4 968 451)
- les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896)
- les polyesters-polyuréthanes obtenus par réaction d'un polyesters de masse moléculaire en nombre de 300-4000 obtenus à partir d'acide adipique et/ou d'acide téréptalique et/ou d'acide sulfoisophtalique et d'un diol de masse inférieure à 300, sur un prépolymère à groupements isocyanates terminaux obtenus à partir d'un polyoxyéthylène glycol de masse moléculaire de 600-4000 et d'un diisocyanate ((FR-A-2 334 698)
- les monoamines ou polyamines éthoxylées, les polymères d'amines éthoxylées (US-A-4 597 898, EP-A-11 984)
- les oligomères polyesters sulfonés obtenus par condensation de l'acide isophtalique, du sulfosuccinate de diméthyle et de diéthylène glycol (FR-A-2 236 926)

Les performances des compositions cosmétiques faisant l'objet de l'invention peuvent aussi être améliorées par l'emploi d'agents plastifiants. L'agent plastifiant pourra constituer entre 0,1 à 20 % de la formulation de préférence de 1 à 15 %. Parmi les agents plastifiants particulièrement utiles, on peut citer les adipates, les phtalates, les isophtalates, les azélates, les stéarates, les copolyols silicones, les glycols, l'huile de ricin, ou leurs mélanges.

On peut aussi avantageusement ajouter à ces compositions des agents séquestrants des métaux, plus particulièrement ceux séquestrants du calcium comme les ions citrates ou des agents émollients comme les silicones ou des huiles ou corps gras utilisés à ce propos dans l'industrie cosmétique (huiles minérales, esters d'acides gras, triglycérides, silicones, ....).

On peut aussi rajouter des polymères hydrosolubles ou hydrodispersables comme le collagène ou certains dérivés non allergisants de protéines animales ou végétales (hydrolisats de protéines de blé par exemple), des hydrocolloïdes naturels (gomme de guar, de caroube, de tara, .....) ou issus de procédés de fermentation comme la gomme xanthane et les dérivés de ces polycarbohydrates comme les celluloses modifiées (par exemple Hydroxyéthylcellulose, carboxyméthylcellulose), les dérivés du guar ou de la caroube comme leurs dérivés cationiques ou des dérivés non-ioniques (par exemple hydroxypropylguar), les dérivés anioniques (carboxyméthylguar et carboxyméthylhydroxypropylguar).

A ces composés, on peut ajouter en association des poudres ou des particules minérales comme du carbonate de calcium, des oxydes minéraux sous forme de poudre ou sous forme colloidale (particules de taille inférieure ou de l'ordre de un micromètre, parfois de quelque dizaines de nanomètres) comme de la silice, des sels d'aluminium utilisés généralement comme antitranspirants, du kaolin, du talc, des argiles et leurs dérivé, ...

A ces ingrédients on rajoute généralement pour augmenter l'agrément lors de l'utilisation de la composition par le consommateur, un ou des parfums, des agents colorants parmi lesquels on peut citer les produits décrits dans l'annexe IV ("list of colouring agents allowed for use in cosmetic products") de la directive européenne n° 76/768/CEE du 27 juillet 1976 dite directive cosmétique et/ou des agents opacifiants comme des pigments.

Finalement, la composition peut aussi contenir des polymères viscosants ou gélifiants, comme les polyacrylates réticulés -CARBOPOL commercialisés par GOODRICH-, les dérivés de la cellulose comme l'hydroxypropylcellulose, la carboxyméthylcellulose, les guars et leurs dérivés, la caroube, la gomme de tara ou de cassia, la gomme xanthane, les alginates, les carraghénannes, les dérivés de la chitine comme le chitosan utilisés seuls ou en association, ou les mêmes composés, généralement sous la fome de polymères hydrosolubles mdifiés par des groupements hydrophobes liés de manière covalente au squelette polymère comme décrit dans le brevet WO 92/16187 et/ou de l'eau pour amener le total des constituants de la formulation à 100 %.

Les compositions cosmétiques faisant l'objet de l'invention peuvent également contenir des agents dispersants polymériques en quantité de l'ordre de 0,1 à 7% en poids, pour contrôler la dureté en calcium et magnésium, agents tels que :

- les sels hydrosolubles d'acides polycarboxyliques de masse moléculaire de l'ordre de 2000 à 100 000, obtenus par polymérisation ou copolymérisation d'acides carboxyliques éthyléniquement insaturés tels que acide acrylique, acide ou anhydride maleique, acide fumarique, acide itaconique, acide aconitique, acide mesaconique, acide citraconique, acide méthylènemalonique , et tout particulièrement les polyacrylates de masse moléculaire de l'ordre de 2 000 à 10 000 (US-A-3 308 067), les copolymères d'acide arylique et d'anhydride maleique de masse moléculaire de l'ordre de 5 000 à 75 000 (EP-A-66 915)

. les polyéthylèneglycols de masse moléculaire de l'ordre de 1000 à 50 000.

L'invention concerne également une composition pour revêtement comprenant les particules selon l'invention et au moins un liant.

De préférence, ces compositions de revêtement sont transparentes telles que, par exemple, les compositions pour lasure ou vernis. Par revêtement transparent, on entend un revêtement à travers lequel le support traité peut être vu. Il peut être coloré ou non.

Cette composition de revêtement peut être à base d'un solvant aqueux ou organique.

Lorsque le solvant est organique, il peut être choisi parmi :

- les hydrocarbures aliphatiques (type white spirit), aromatiques (type xylène) ou terpéniques (type essence de térébentine),
- les acétates (type acétate de butyle),
- les cétones (type acétone),
- les alcools (type butanol),

ou de mélanges de ces solvants.

Les particules de dioxyde de titane, sous forme de dispersion ou de poudre, présentent les caractéristiques définies précédemment.

Le liant peut être choisi parmi les polymères hydrosolubles, les latex type alkyde, arninoplastes, acrylique ou styrène/acrylique, vinylique, cellulosique, polyuréthanne, époxy, ... Ces liants conviennent aussi bien pour des compositions de revêtement en phase aqueuse qu'en phase organique.

Selon une variante préférée, on peut utiliser des latex acryliques de petite taille, i.e. de taille inférieure à 100 nm, tels que les latex RHODOPAS ULTRAFINE® commercialisés par Rhône-Poulenc, notamment en lasure.

La composition peut comprendre également un agent tensio-actif.

L'agent tensio-actif est de préférence un agent tensio-actif non ionique par exemple éthoxylé avec un nombre d'éthoxylation compris entre 3 et 12, par exemple un agent tensio-actif propoxylé (OP). La quantité d'agent tensio-actif peut être comprise entre 1 et 120 % en poids par rapport au dioxyde de titane exprimé en sec.

Cette composition peut comprendre tout type d'agents classiquement utilisés dans l'application revêtement tels que des épaississants, des agents biocides, des agents anti-UV (HALS ou absorbeurs organiques), des pigments colorants, des agents de coalescence.

En général, la teneur de ladite composition en particules de dioxyde de titane est telle que la teneur du revêtement sec, obtenu à partir de la composition, en dioxyde de titane est d'au plus 10 % en poids, de préférence comprise entre 1 et 8 %.

Cette composition peut notamment être utilisée pour tout revêtement transparent, notamment en lasure ou en vernis.

Elles sont particulièrement stables au stockage et on peut constater que les particules de dioxyde de titane conserve leur indice de dispersion dans la formulation.

Les exemples suivants illustrent l'invention sans toutefois en limiter la portée.

## EXEMPLES

### Exemple 1 - Préparation d'une dispersion 1 selon l'invention avec un traitement à base de $CeO_2$ et $SiO_2$

Etape 1 - Hydrolyse

On ajoute successivement à 394,7 g d'une solution d'oxychlorure de titane à 1,9 mol/kg :

- 42,02 g d'acide chlorhydrique à 36 %,
- 4,73 g d'acide citrique,
- 547,1 g d'eau épurée,
- 11,36 g (0,2 % /$TiO_2$) de germes d'anatase présentant une taille compris entre 5 et 6 nm.

Le mélange est porté à ébullition est y est maintenu pendant 3 h.

Etape 2 - Récupération des particules et remise en dispersion

La solution est ensuite filtrée et les particules obtenues sont lavées à l'eau jusqu'à élimination complète des chlorures. Elles sont ensuite redispersées à pH 9 (contrôlé par l'ajout de soude). L'extrait sec est de 20 % en poids.
La dispersion obtenue est stable. La taille des particules mesurée par MET est de 45 nm. L'analyse par diffraction X indique que les particules sont à base de dioxyde de titane uniquement sous forme anatase.
Leur densité est de 2,5 (Vi = 0,14 cc/g).
La surface spécifique, mesurée par la méthode BET sur les particules de la dispersion séchées à une température de dégazage de 150 °C, est de 300 $m^2$/g.

Etape 3 - Traitement des particules par un composé du cérium

On utilise une solution 1 qui est une solution aqueuse d'acétate de cérium à 3,3 % en poids de CeO2.
On introduit 2822 g de la dispersion obtenue à l'issue de l'étape 2 dans un réacteur muni d'une agitation. Puis, on ajoute 5000 g d'eau épurée. Le pH de la dispersion est ajusté à 9,1 par ajout de soude. La température est maintenue à 25°C.
On introduit ensuite 456 g de la solution 1 de façon continue avec un débit de 9,5 g/min. Le pH est régulé à 9 par introduction simultanée d'une solution aqueuse de soude à 10%.
On effectue ensuite un mûrissement de 15 min sous agitation.

Etape 4 - Traitement des particules par un composé du silicium

On utilise la solution 2 qui est une solution aqueuse de silicate de sodium à 26,2 % en poids de SiO2.
Le milieu réactionnel issu de l'étape 3 est chauffé à 90 °C (montée en température en 1h30 min) et le pH régulé à 8.
On introduit 381 g de la solution 2 avec un débit de 6,3 g/min, puis on coupe le chauffage et on laisse refroidir à température ambiante.
On introduit 125 ml de $H_2SO_4$ 3 M, le pH est de 5,5.
La dispersion obtenue est lavée par ultrafiltration.

Résultats

L'extrait sec de la dispersion est de 25 % en poids.
La taille des particules mesurée par MET est de 45 nm.
Le taux de cérium, mesuré par fluorescence X, est de 4,5 % en poids, exprimé en $Ce_2O$, mesuré par fluorescence X, par rapport au $TiO_2$, le taux de $SiO_2$ est de 30 % en poids.
La surface spécifique, mesurée par la méthode BET sur les particules de la dispersion séchées sous vide à une température de 150 °C pendant 4 heures, est de 100 $m^2$/g.
La densité est de 2,48.
La viscosité est de 400 mPa.s.
L'indice de dispersion est de 0,4. Cette dispersion est particulièrement stable : au bout d'un mois, la mesure de l'indice de dispersion reste de 0,4.

**Exemple 2 - Préparation d'une dispersion 2 selon l'invention avec traitement de surface à base de $CeO_2$, $SiO_2$ et $Al_2O_3$**

Etape 1 - Hydrolyse

On ajoute successivement à 394,7 g d'une solution d'oxychlorure de titane à 1,9 mol/kg :

- 42,02 g d'acide chlorhydrique à 36 %,
- 4,73 g d'acide citrique,
- 547,1 g d'eau épurée,
- 5,68 g (0,1 % /$TiO_2$) de germes d'anatase présentant une taille compris entre 5 et 6 nm.

Le mélange est porté à ébullition est y est maintenu pendant 3 h.

Etape 2 - Récupération des particules et remise en dispersion

La solution est ensuite filtrée et les particules obtenues sont lavées à l'eau jusqu'à élimination complète des chlorures. Elles sont ensuite redispersées à pH 9 (contrôlé par l'ajout de soude) avec un extrait sec est de 20 % en poids.
La dispersion obtenue est stable. La taille des particules mesurée par MET est de 60 nm. L'analyse par diffraction X indique que les particules sont à base de dioxyde de titane uniquement sous forme anatase.
Leur densité est de 2,52 (Vi = 0,14 cc/g).
La surface spécifique, mesurée par la méthode BET sur les particules de la dispersion séchées à une température de dégazage de 150 °C, est de 300 $m^2/g$.

Etape 3 - Traitement des particules par un composé du cérium

On utilise une solution 4 qui est une solution aqueuse d'acétate de cérium obtenue par dissolution de 13,56 g d'acétate de cérium dans 750 g d'eau.
On introduit 1159 g de la dispersion obtenue à l'issue de l'étape 2 dans un réacteur de 3 litres muni d'une agitation. Puis, on ajoute 240 g d'eau épurée. Le pH de la dispersion est ajusté à 9 par ajout de soude. La température est maintenue à 25 °C.
On introduit ensuite la solution 4 de façon continue avec un débit de 500 ml/h. Le pH est régulé à 9 par introduction simultanée d'une solution aqueuse de soude 5 M et d'une solution aqueuse d'acide phosphorique 1 M. On effectue ensuite un mûrissement de 1 heure à 25 °C, toujours sous agitation.

Etape 4 - Traitement des particules par de la silice et de l'alumine

On utilise les solutions suivantes :

- solution 2 : solution aqueuse de silicate de cérium obtenue par dissolution de 108,78 g de silicate de sodium dans 100 g d'eau (silicate de sodium à 355 g/l de $SiO_2$ et rapport molaire $SiO_2/Na_2O$ = 3,3)
- solution 3 : solution aqueuse d'aluminate de sodium obtenue par dissolution de 39,28 g d'aluminate de sodium dans 40 g d'eau (aluminate de sodium à 24 % d'$Al_2O_3$ et 19 % de $Na_2O$)

Le milieu réactionnel est ensuite chauffé à 90 °C (montée en température en 35 min) et la solution 2 est introduite avec un débit de 2 ml/min. Le pH est régulé à 9 par l'introduction de $H_2SO_4$ 3M. On effectue un temps de mûrissement de 1 heure à 90 °C.
Toujours à 90 °C et à pH 9, on introduit la solution 3 avec un débit de 2 ml/min, puis on laisse mûrir 1 heure. Le milieu réactionnel est ensuite refroidi à température ambiante.

Résultats

La dispersion obtenue est centrifugée. Le gâteau obtenu est lavé avec de l'eau épurée puis ce gâteau est redispersé en milieu aqueux à pH 9.
L'extrait sec de la dispersion est de 25 % en poids.
La taille des particules mesurée par MET est de 60 nm. Le taux de cérium est de 5 % en poids, exprimé en $Ce_2O$ par rapport au $TiO_2$, le taux de $SiO_2$ est de 15 % en poids et celui de $Al_2O_3$ de 5 %.
La surface spécifique, mesurée par la méthode BET sur les particules de la dispersion séchées sous vide à une température de 150 °C pendant 4 heures, est de 120 $m^2/g$.
La densité est de 2,50.
La viscosité est de 500 mPa.s.
L'indice de dispersion est de 0,4. Cette dispersion est particulièrement stable : au bout d'un mois, la mesure de l'indice de dispersion reste de 0,4.

**Exemple 3 - préparation d'une dispersion 3 selon l'invention avec un traitement à base de $CeO_2$, $SiO_2$, $Al_2O_3$**

On met en oeuvre les étapes 1 et 2 de l'exemple 1.

Etape 3 - Traitement des particules par un composé du cérium

On utilise une solution 1 qui est une solution aqueuse d'acétate de cérium obtenue par dissolution de 13,56 g d'acétate de cérium dans 750 g d'eau épurée.

On introduit 1159 g de la dispersion obtenue à l'issue de l'étape 2 dans un réacteur de 3 litres muni d'une agitation. Puis, on ajoute 240 g d'eau épurée. Le pH de la dispersion est ajusté à 9 par ajout de soude. La température est maintenue à 25 °C.

On introduit ensuite la solution 1 de façon continue avec un débit de 2 ml/min. Le pH est régulé à 9 par introduction simultanée d'une solution aqueuse de soude 5 M et d'une solution aqueuse d'acide phosphorique 1 M. On effectue ensuite un mûrissement de 1 heure à 25 °C, toujours sous agitation.

Etape 4 - Traitement des particules par des composés du silicium et de l'aluminium

On utilise les solutions suivantes :

- solution 2 : solution aqueuse de silicate de sodium obtenue par dissolution de 108,78 g de silicate de sodium dans 100 g d'eau (silicate de sodium à 355 g/l de $SiO_2$ et rapport molaire $SiO_2/Na_2O$ = 3,3)
- solution 3 : solution aqueuse d'aluminate de sodium obtenue par dissolution de 39,28 g d'aluminate de sodium dans 40 g d'eau (aluminate de sodium à 24 % en poids d'$Al_2O_3$ et 19 % en poids de $Na_2O$)

Le milieu réactionnel issu de l'étape 3 est chauffé à 90 °C (montée en température en 35 min) et la solution 2 est introduite avec un débit de 2 ml/min. Le pH est régulé à 9 par l'introduction de $H_2SO_4$ 3 M. On effectue un temps de mûrissement de 1 heure à 90 °C.

Toujours à 90 °C et à pH 9, on introduit la solution 3 avec un débit de 2 ml/min, puis on laisse mûrir 1 heure. Le milieu réactionnel est ensuite refroidi à température ambiante.

Résultats

La dispersion obtenue est centrifugée. Le gâteau obtenu est lavé avec de l'eau épurée, puis ce gâteau est redispersé en milieu aqueux à pH 9.

L'extrait sec de la dispersion est de 25 % en poids.

La taille des particules mesurée par MET est de 45 nm. Le taux de cérium est de 5 % en poids, exprimé en $CeO_2$ par rapport au $TiO_2$, le taux de $SiO_2$ est de 15,75 % en poids par rapport au $TiO_2$ et celui de $Al_2O_3$ de 5,25 % par rapport au $TiO_2$.

La surface spécifique, mesurée par la méthode BET sur les particules de la dispersion séchées sous vide à une température de 150 °C pendant 4 heures, est de 100 $m^2$/g.

La densité est de 2,48.

La viscosité est de 200 mPa.s.

L'indice de dispersion est de 0,4. Cette dispersion est particulièrement stable : au bout d'un mois, la mesure de l'indice de dispersion reste de 0,4.

**Exemple 4 - préparation cosmétique anti-UV**

On prépare une composition cosmétique anti-UV selon l'invention à partir de la dispersion de dioxyde de titane de l'exemple 3 en suivant la formulation suivante :

| CTFA | Ingrédients | % en poids |
|---|---|---|
| cyclométhicone et diphényldiméthicone | Mirasil C-DPDM | 4 |
| caprylic capric triglycéride | Miglyol 812 N | 4 |
| palmitate d'octyl | Crodamol OP | 4 |
| huile minérale | Marcol 82 | 5 |
| copolymère PVP/eicosène | Antaron V 220 | 3 |
| acétate de vitamine E | | 0,3 |
| stéarate de glycéryl | | |
| stéarate de propylème glycol | | |
| isostéarate de glycéryl | Hydrolactol 70 | 10 |
| isostéarate de propylème glycol | | |
| olleth 25 | | |
| ceteth 25 | | |

(suite)

| CTFA | Ingrédients | % en poids |
|---|---|---|
| cecyl phosphate de potassium | Amphisol K | 2 |
| polysorbate 20 | Tween 20 | 1 |
| conservateur | Germaben II | 0,2 |
| allantoine | | 0,2 |
| gomme xanthane | Rhodicare D | 0,2 |
| dispersion exemple 2 | | 15,6 (5% en TiO2) |
| acide lactique | | qs pH = 6,5 |
| eau déionisée | | qs 100 |

Test de ohotostabilité

La composition cosmétique à tester est introduite via une cellule en quartz dans un appareil Sun-Test de Heraeus et soumise à une énergie E de 500 W/m$^2$ à une température T de 30 °C durant 1 h.

Le contrôle de la photostabilité se fait par observation visuelle de la coloration de la formulation. Visuellement, on n'observe aucun bleuissement.

Mesure de l'indice SPF (Sun Protection Factor) in vitro

Cet indice a été mesuré à l'aide d'un appareil SPF290 d'optométrie selon la méthode décrite dans "Cosmetics & Toiletries", Vol.107, N°10, p.119.

L'indice SPF est 20 ±2 pour une application de 2 mg/cm$^2$.

Mesure de l'indice de dispersion des particules de titane dans la formulation

L'indice de dispersion est 0,4, c'est-à-dire identique à celui de la dispersion de particules de dioxyde de titane avant formulation.

**Exemple 5 - évaluation des propriétés des lasures**

Préparation des lasures à partir de dispersions de particules de dioxyde de titane

On mélange les composants suivants :

- 407,9 g d'eau,
- 3,7 g de Thixol® 60 commercialisé par COATEX qui est un épaississant,
- 2 g de Clérol® TPE 714 commercialisé par BEVALOID qui est un agent anti-mousse,
- 20 g de Dowanol® dpnb commercialisé par DOW qui est un agent de coalescence,
- 20 g de propylène glycol qui est un agent de coalescence,
- 523 g de DS 913 commercialisé par Rhône-Poulenc qui est un latex styrène/acrylique,
- 3,3 g de Proxel® gxl qui est un agent biocide,
- 3,4 g de NaOH à 30 %.

Le mélange est effectué à l'aide d'un agitateur Rayneri, puis on y ajoute 55,06 g d'une dispersion à tester.

Mesure de la transparence

Un film de lasure d'une épaisseur de 100 µm humide est tiré sur un carton LENETA (fond noir et blanc), donnant un film sec d'une épaisseur de 20 µm.

La transparence est évaluée à l'aide d'un spectrophotomètre DATACOLOR DC 3890 en mode spéculaire inclus. Le résultat est exprimé en ΔL (différence entre le L du fond noir traité par la lasure et d'un fond noir non traité). Plus ce ΔL est faible, meilleure est la transparence.

L est la mesure de la réflectance (nuance clair/sombre) dans la coloration intrinsèque quantifiée au moyen des coordonnées chromatiques L, a et b, données dans le système CIE 1976 (L, a, b) tel que défini par la Commission

Internationale d'Eclairage et répertorié dans la Recueil des Normes Françaises (AFNOR), couleur colorimétrique n° X08-12 (1983).

Mesure de l'activité anti-UV

On applique 3 couches de lasure à raison de 300 g/$m^2$ sur des panneaux de bois (pin maritime 15 X 18 X 1 $cm^3$). Ces panneaux sont exposés à des rayons UV dans un appareil de vieillissement accéléré QUVB commercialisé par la société Q-PANEL fonctionnant à 60 °C.

La protection anti-UV peut être évaluée à la fois par une appréciation visuelle et une mesure colorimétrique à travers l'évolution du facteur de réflectance L tel que défini précédemment. En effet, au cours du vieillissement accéléré, plus la chute de L est forte, plus le bois grisaille et vieillit.

Le résultat a été exprimé en $\Delta L$ qui est la différence entre le L initial du bois et le L du bois après vieillissement accéléré.

On observe pour la lasure ne contenant pas de particules de dioxyde de titane présente un $\Delta L$ de 12 ; la couleur après vieillissement est très hétérogène (écaillage).

Mesure de l'activité photocatalytique

L'activité photocatalytique est évaluée sur les particules de dioxyde de titane sous forme de poudre. Pour cela, les dispersions de dioxyde de titane sont séchées dans une étuve à 120 °C.

Le test consiste à mesurer la vitesse de photooxydation du gaz isopropanol par le dioxyde de titane selon un test de même principe que celui décrit dans l'article de Irick G., Strickland T.H. and Zanucci J.S., Permanence of Organic Coatings ASTM STP 781, 1982, p. 35-42.

On introduit dans un tube en Pyrex 3 ml d'isopropanol et 1,5 g de dioxyde de titane à tester. Ce dernier est immobilisé sous forme d'une pastille sur un support métallique ; la surface de dioxyde de titane exposée aux UV est de 10 $cm^2$. Le tube est ensuite scellé.

L'appareil de test est constitué d'un carrousel tournant autour de 3 lampes UVA basse pression présentant un maximum d'émission entre 300 et 400 nm. Le tube contenant l'échantillon est placé dans le carrousel, la face du support contenant le dioxyde de titane à évaluer du côté du rayonnement UVA. Le carrousel est lui-même placé dans une étuve à 60 °C.

La puissance lumineuse dans l'UVA reçue par le dioxyde de titane est d'environ 30 W/$m^2$.

L'isopropanol en phase vapeur est adsorbé sur le dioxyde de titane et réagit avec l'oxygène pour former de l'acétone.

On dose régulièrement sur une période de 24 heures à l'aide d'un chromatographe en phase gazeuse l'avancement de la photodécomposition de l'isopropanol par suivi de la quantité d'$O_2$ restant dans le tube. On traduit cet avancement à l'aide la constante de vitesse de disparition de l'$O_2$ exprimée en mmole/h/$m^2$.

Plus la vitesse de photooxydation mesurée par ce test est forte, plus la dégradation de la lasure sera importante.

Les résultats de ce test ont été confirmés par une évaluation de l'aspect visuel de la lasure après 200 cycles de vieillissement accéléré tel que précédemment décrit. Selon l'activité photocatalytique, on peut observer un farinage plus ou moins prononcé.

La dispersion 3 est testée ainsi qu'une dispersion 4 de l'art antérieur qui est une dispersion de particules de dioxyde de titane rutile présentant un extrait sec de 25 % en poids. Les particules de cette dispersion 4 présentant une taille moyenne de 80 nm et un traitement de surface à base de silice dans une teneur de 110 % en poids.

Résultats

| | | | Activité photocatalytique | | |
|---|---|---|---|---|---|
| | | | Vitesse disparition $O_2$ (mmole/h/m²) | Aspect du film | |
| Dispersion | Transparence ΔL | Activité anti-UV : ΔL après 20 cycles | | à l'état initial | après 200 cycles |
| Dispersion 3 | 4,3 | 6 | 0,23 | homogène | un peu de farinage |
| Dispersion 4 | 5,7 | 8 | 0,33 | hétérogène | farinage |

On observe que les dispersions de particules de dioxyde de titane traitée par du cérium présentent une faible activité photocatalytique. Ceci permet d'éviter le farinage de la lasure et de conserver à cette lasure son aspect initial tout en assurant une protection anti-UV.

De plus, ces lasures présentent une bonne transparence.

## Revendications

1. Particules de dioxyde de titane anatase de taille d'au plus 100 nm, **caractérisées en ce que** ces particules sont recouvertes au moins partiellement :

   - d'une première couche d'au moins un composé du cérium et/ou du fer, et
   - d'une deuxième couche d'au moins un oxyde, hydroxyde ou oxohydroxyde métallique,

   et **en ce que** ces particules présentent une surface spécifique BET d'au moins 70 $m^2$/g et une densité de l'ordre de 2,5.

2. Particules selon la revendication 1, **caractérisées en ce que** la première couche est à base d'au moins un composé du cérium dans une teneur telle que le rapport en poids du composé du cérium sur le dioxyde de titane est d'au plus 6 % en poids.

3. Particules selon la revendication 1 ou 2, **caractérisées en ce que** la deuxième couche est à base de silice et/ou d'un hydroxyde ou oxohydroxyde d'aluminium sous forme simple ou mixte.

4. Particules selon la revendication 3, **caractérisées en ce que** la deuxième couche est à base d'une couche de silice dans une teneur en poids de 30 % de $SiO_2$ par rapport au dioxyde de titane.

5. Particules selon l'une quelconque des revendications précédentes, **caractérisées en ce qu'**elles se présentent sous forme de dispersion.

6. Particules selon la revendication 5, **caractérisées en ce que** leur indice de dispersion dans la phase liquide est d'au plus 0,5.

7. Particules selon la revendication 5 ou 6, **caractérisées en ce que** la phase liquide de la dispersion est aqueuse.

8. Particules selon la revendication 7, **caractérisées en ce que** la dispersion présente une conductivité d'au plus 3 msiemens.

9. Particules selon l'une quelconque des revendications 7 ou 8, **caractérisées en ce que** l'extrait sec de la dispersion est d'au moins 35 % en poids et **en ce que** la viscosité de ladite dispersion est d'au plus 1000 mPa.s.

**10.** Particules selon la revendication 5 ou 6, **caractérisées en ce que** la phase liquide de la dispersion est organique.

**11.** Particules selon la revendication 10, **caractérisées en ce que** la dispersion organique est obtenue par mélange de particules en dispersion aqueuse selon l'une des revendications 7 à 9 avec un milieu organique comprenant un agent de transfert, ce dernier étant choisi parmi les amines quaternaires ou les sels d'ammonium quaternaire.

**12.** Particules selon l'une des revendications 1 à 5, **caractérisées en ce qu'**elles sont agglomérées et se présentent sous forme d'une poudre.

**13.** Procédé de préparation de particules selon l'une des revendications 1 à 12, **caractérisé en ce qu'**on met en oeuvre les étapes suivantes :

- on précipite au moins un composé du cérium et/ou du fer à la surface de particules de dioxyde de titane anatase de taille d'au plus 100 nm, présentant une surface spécifique BET d'au moins 200 $m^2/g$ et une densité de l'ordre de 2,5, puis
- on précipite au moins un oxyde, hydroxyde ou oxohydroxyde métallique à la surface de particules obtenues.

**14.** Procédé de préparation selon la revendication 13, **caractérisé en ce que** les particules de dioxyde de titane de départ sont obtenues par hydrolyse d'au moins un composé du titane A en présence d'au moins un composé B choisi parmi :

(i) les acides qui présentent :

- soit un groupement carboxyle et au moins deux groupements hydroxyles et/ou amines,
- soit au moins deux groupements carboxyles et au moins un groupement hydroxyle et/ou amine,

(ii) les acides phosphoriques organiques de formules suivantes :

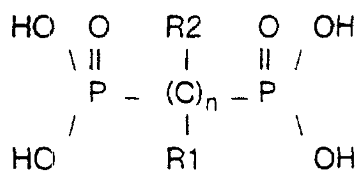

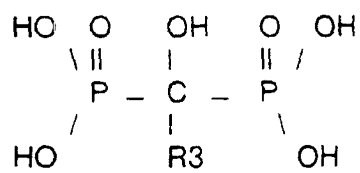

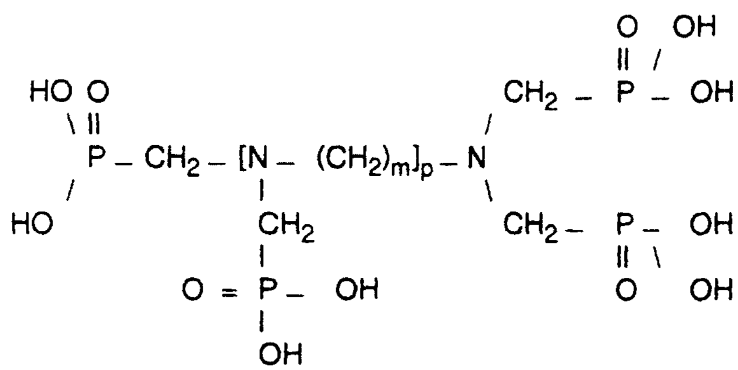

dans lesquelles, n et m sont des nombres entiers compris entre 1 et 6, p est un nombre entier compris entre 0 et 5, R1, R2, R3 identiques ou différents représentant un groupement hydroxyle, amino, aralkyl, aryl, alkyl ou l'hydrogène,
(iii) les composés capables de libérer des ions sulfates en milieu acide,
(iv) les sels des acides décrits ci-dessus,

et en présence de germes de dioxyde de titane anatase présentant une taille d'au plus 5 nm et dans un rapport pondéral exprimé en TiO2 présent dans les germes/titane présent avant introduction des germes dans le milieu d'hydrolyse, exprimé en $TiO_2$ compris entre 0,01 % et 3 %.

**15.** Procédé selon la revendication 14, **caractérisé en ce que** le composé du titane A est l'oxychlorure de titane.

**16.** Procédé selon la revendication 14 ou 15, **caractérisé en ce que** le composé B est l'acide citrique.

**17.** Utilisation des particules selon l'une des revendications 1 à 12 en tant qu'agent anti-UV.

**18.** Utilisation selon la revendication 17 dans les formulations pour cosmétiques, revêtements et dans les plastiques.

**19.** Composition cosmétique anti-UV, **caractérisée en ce qu'**elle comprend des particules de dioxyde de titane selon l'une des revendications 1 à 12, en quantité telle que la teneur de ladite composition en dioxyde de titane est d'au moins 1 %, de préférence d'au plus 25 % en poids.

**20.** Composition de revêtement anti-UV, **caractérisée en ce qu'**elle comprend au moins un liant et des particules de dioxyde de titane selon l'une des revendications 1 à 12, en quantité telle que la teneur de ladite composition en dioxyde de titane est d'au plus 10 % en poids.

**21.** Composition de revêtement anti-UV selon la revendication 20, **caractérisée en ce qu'**elle comprend un agent tensio-actif choisi parmi les agents tensio-actifs non ioniques éthoxylés présentant un nombre d'éthoxylation compris entre 3 et 12.

**Patentansprüche**

**1.** Anatas-Titandioxid-Teilchen mit einer Grösse von höchstens 100 nm, **dadurch gekennzeichnet, dass** diese Teilchen mindestens teilweise bedeckt sind:

- von einer ersten Schicht von mindestens einer Cer- und/oder Eisenverbindung und
- von einer zweiten Schicht von mindestens einem Metalloxid, -hydroxid oder -oxohydroxid

und dass diese Teilchen eine spezifische BET-Oberfläche von mindestens 70 $m^2/g$ und eine Dichte in der Grössenordnung von 2,5 aufweisen.

**2.** Teilchen nach Anspruch 1, **dadurch gekennzeichnet, dass** die erste Schicht auf mindestens einer Cerverbindung mit einem solchen Gehalt, dass das Gewichtsverhältnis der Cerverbindung zu dem Titandioxid höchstens 6 Gew.-% beträgt, basiert.

**3.** Teilchen nach Anspruch 1 oder 2, **dadurch gekennzeichnet, dass** die zweite Schicht auf Siliciumdioxid und/oder einem Aluminiumhydroxid oder -oxohydroxid in einfacher oder gemischter Form basiert.

**4.** Teilchen nach Anspruch 3, **dadurch gekennzeichnet, dass** die zweite Schicht auf einer Siliciumdioxidschicht mit einem 30 Gew.-%-igen Gehalt an $SiO_2$ bezogen auf das Titandioxid basiert.

**5.** Teilchen nach einem der vorangegangenen Ansprüche, **dadurch gekennzeichnet, dass** sie in Form einer Dispersion vorliegen.

**6.** Teilchen nach Anspruch 5, **dadurch gekennzeichnet, dass** ihr Dispersionsindex in der flüssigen Phase höchstens 0,5 beträgt.

**7.** Teilchen nach Anspruch 5 oder 6, **dadurch gekennzeichnet, dass** die flüssige Phase der Dispersion wässrig ist.

**8.** Teilchen nach Anspruch 7, **dadurch gekennzeichnet, dass** die Dispersion eine Leitfähigkeit von höchstens 3 Millisiemens aufweist.

**9.** Teilchen nach einem der Ansprüche 7 oder 8, **dadurch gekennzeichnet, dass** der Trockenauszug der Dispersion mindestens 35 Gew.-% beträgt und dass die Viskosität der Dispersion höchstens 1000 mPa·s beträgt.

**10.** Teilchen nach Anspruch 5 oder 6, **dadurch gekennzeichnet, dass** die flüssige Phase der Dispersion organisch ist.

**11.** Teilchen nach Anspruch 10, **dadurch gekennzeichnet, dass** die organische Dispersion durch Mischen von Teilchen in wässriger Dispersion nach einem der Ansprüche 7-bis 9 mit einem organischen Medium, das ein Transfermittel umfasst, wobei dieses letztere unter den quartären Aminen oder den quartären Ammoniumsalzen ausgewählt wird, erhalten wird.

**12.** Teilchen nach einem der Ansprüche 1 bis 5, **dadurch gekennzeichnet, dass** sie agglomeriert sind und in Form eines Pulvers vorliegen.

**13.** Verfahren zur Herstellung von Teilchen nach einem der Ansprüche 1 bis 12, **dadurch gekennzeichnet, dass** man die folgenden Schritte ausführt:

- man präzipitiert mindestens eine Cer- und/oder Eisenverbindung auf der Oberfläche von Anatas-Titandioxid-Teilchen mit einer Grösse von höchstens 100 nm, die eine spezifische BET-Oberfläche von mindestens 200 $m^2/g$ und eine Dichte in der Grössenordnung von 2,5 aufweisen, dann
- präzipitiert man mindestens ein Metalloxid, -hydroxid oder -oxohydroxid auf der Oberfläche von erhaltenen Teilchen.

**14.** Herstellungsverfahren nach Anspruch 13, **dadurch gekennzeichnet, dass** die als Ausgangsmaterial verwendeten Titandioxidteilchen erhalten werden durch Hydrolyse von mindestens einer Titanverbindung A in Gegenwart von mindestens einer Verbindung B, ausgewählt unter:

(i) den Säuren, die aufweisen:

- entweder eine Carboxylgruppe und mindestens zwei Hydroxyl- und/oder Amingruppen,
- oder mindestens zwei Carboxylgruppen und mindestens eine Hydroxyl- und/oder Amingruppe,

(ii) den organischen Phosphorsäuren mit den folgenden Formeln:

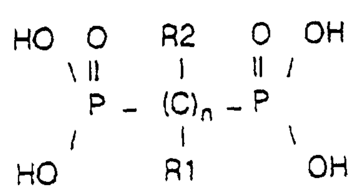

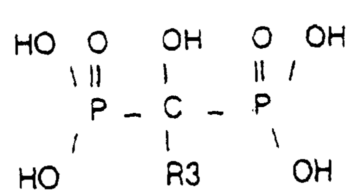

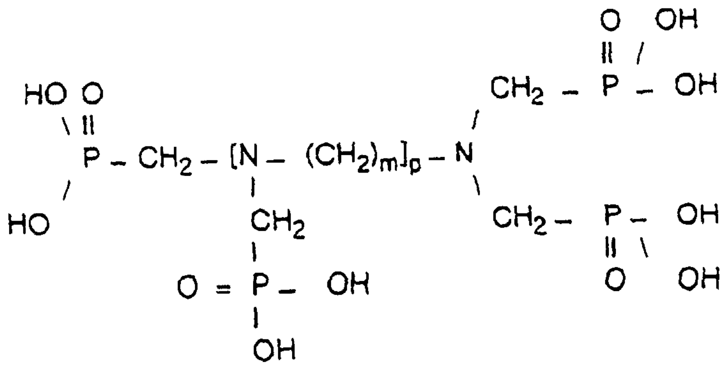

in denen n und m ganze Zahlen zwischen 1 und 6 sind, p eine ganze Zahl zwischen 0 und 5 ist, R1, R2, R3, die gleich oder unterschiedlich sind, für eine Hydroxyl-, Amino-, Arylalkyl-, Aryl-, Alkylgruppe oder Wasserstoff stehen,

(iii) den Verbindungen, die in der Lage sind, in saurem Milieu Sulfationen freizusetzen,

(iv) den Salzen der oben beschriebenen Säuren, und in Gegenwart von Anatas-Titandioxid-Keimen, die eine Grösse von höchstens 5 nm aufweisen, in einem Gewichtsverhältnis, ausgedrückt in $TiO_2$, das in den Keimen vorhanden ist, /Titan, das vor Hinzufügen der Keime in dem Hydrolysemedium vorhanden ist, ausgedrückt in $TiO_2$, zwischen 0,01% und 3%.

**15.** Verfahren nach Anspruch 14, **dadurch gekennzeichnet, dass** die Titanverbindung A Titanoxychlorid ist.

**16.** Verfahren nach Anspruch 14 oder 15, **dadurch gekennzeichnet, dass** die Verbindung B Citronensäure ist.

**17.** Verwendung der Teilchen nach einem der Ansprüche 1 bis 12 als Anti-UV-Mittel.

**18.** Verwendung nach Anspruch 17 in Formulierungen für Kosmetika, Überzüge und in Kunststoffen.

**19.** Kosmetische Anti-UV-Zusammensetzung, **dadurch gekennzeichnet, dass** sie Titandioxidteilchen nach einem der Ansprüche 1 bis 12 in einer solchen Menge, dass der Gehalt der Zusammensetzung an Titandioxid mindestens 1 Gew.-%, vorzugsweise höchstens 25 Gew.-% beträgt, umfasst.

**20.** Anti-UV-Überzugszusammensetzung, **dadurch gekennzeichnet, dass** sie mindestens ein Bindemittel und Titandioxidteilchen nach einem der Ansprüche 1 bis 12 in einer solchen Menge, dass der Gehalt der Zusammensetzung an Titandioxid höchstens 10 Gew.-% beträgt, umfasst.

**21.** Anti-UV-Überzugszusammensetzung nach Anspruch 20, **dadurch gekennzeichnet, dass** sie ein grenzflächenaktives Mittel, ausgewählt unter den nicht-ionischen ethoxylierten grenzflächenaktiven Mitteln, die eine Ethoxylierungszahl zwischen 3 und 12 aufweisen, umfasst.

## Claims

**1.** Anatase titanium dioxide particles with a size of at most 100 nm, **characterized in that** these particles are at least partially covered:

- with a first layer of at least one cerium and/or iron compound, and
- with a second layer of at least one metal oxide, hydroxide or hydroxide oxide,

and **in that** these particles exhibit a BET specific surface of at least 70 $m^2/g$ and a relative density of the order of 2.5.

**2.** Particles according to claim 1, **characterized in that** the first layer is based on at least one cerium compound in a content such that the ratio by weight of the cerium compound to the titanium dioxide is at most 6% by weight.

**3.** Particles according to claim 1 or 2,

**characterized in that** the second layer is based on silica and/or on an aluminium hydroxide or hydroxide oxide, in the simple or mixed form.

4. Particles according to claim 3, **characterized in that** the second layer is based on a layer of silica in a content by weight of 30% of $SiO_2$ with respect to the titanium dioxide.

5. Particles according to any one of the preceding claims, **characterized in that** they are provided in the form of a dispersion.

6. Particles according to claim 5, **characterized in that** their dispersion index in the liquid phase is at most 0.5.

7. Particles according to claim 5 or 6,
**characterized in that** the liquid phase of the dispersion is aqueous.

8. Particles according to claim 7, **characterized in that** the dispersion exhibits a conductivity of at most 3 msiemens.

9. Particles according to either one of claims 7 and 8, **characterized in that** the solids content of the dispersion is at least 35% by weight and **in that** the viscosity of the said dispersion is at most 1000 mPa·s.

10. Particles according to claim 5 or 6,
**characterized in that** the liquid phase of the dispersion is organic.

11. Particles according to claim 10,
**characterized in that** the organic dispersion is obtained by mixing particles in an aqueous dispersion according to one of claims 7 to 9 with an organic medium comprising a transfer agent, the latter being chosen from quaternary amines or quaternary ammonium salts.

12. Particles according to one of claims 1 to 5,
**characterized in that** they are agglomerated and are provided in the form of a powder.

13. Process for the preparation of particles according to one of claims 1 to 12, **characterized in that** the following stages are employed:

- at least one cerium and/or iron compound is precipitated at the surface of anatase titanium dioxide particles with a size of at most 100 nm exhibiting a BET specific surface of at least 200 $m^2/g$ and a relative density of the order of 2.5, then
- at least one metal oxide, hydroxide or hydroxide oxide is precipitated at the surface of particles obtained.

14. Preparation process according to claim 13,
**characterized in that** the starting titanium dioxide particles are obtained by hydrolysis of at least one titanium compound A in the presence of at least one compound B chosen from:

(i) the acids which exhibit:

- either a carboxyl group and at least two hydroxyl and/or amine groups,
- or at least two carboxyl groups and at least one hydroxyl and/or amine group,

(ii) the organic phosphoric acids of following formulae:

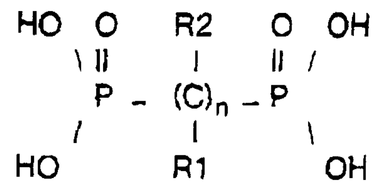

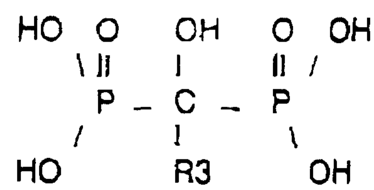

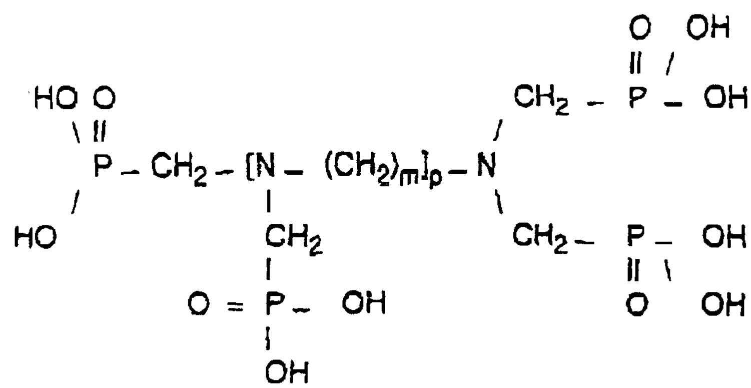

in which n and m are integers of between 1 and 6 and p is an integer of between 0 and 5, R1, R2 and R3, which are identical or different, representing a hydroxyl, amino, aralkyl, aryl or alkyl group or hydrogen,
(iii) the compounds capable of releasing sulphate ions in acidic medium,
(iv) the salts of the acids described above,

and in the presence of anatase titanium dioxide seeds exhibiting a size of at most 5 nm and in a ratio by weight expressed in terms of $TiO_2$ present in the seeds/titanium present before introduction of the seeds in the hydrolysis mixture, expressed in terms of $TiO_2$, of between 0.01% and 3%.

**15.** Process according to claim 14, **characterized in that** the titanium compound A is titanium oxychloride.

**16.** Process according to claim 14 or 15,
**characterized in that** the compound B is citric acid.

**17.** Use of the particles according to one of claims 1 to 12 as anti-UV agent.

**18.** Use according to claim 17 in cosmetic formulations or coating formulations and in plastics.

**19.** Anti-UV cosmetic composition, **characterized in that** it comprises titanium dioxide particles according to one of claims 1 to 12, in an amount such that the content of titanium dioxide in the said composition is at least 1% and preferably at most 25% by weight.

**20.** Anti-UV coating composition, **characterized in that** it comprises at least one binder and titanium dioxide particles according to one of claims 1 to 12, in an amount such that the content of titanium dioxide in the said composition is at most 10% by weight.

**21.** Anti-UV coating composition according to claim 20, **characterized in that** it comprises a surface-active agent chosen from ethoxylated non-ionic surface-active agents exhibiting an ethoxylation number of between 3 and 12.